# EUROPEAN PATENT SPECIFICATION

(11) **EP 1 423 076 B1**
(45) Date of publication and mention of the grant of the patent: **27.05.2009**
(21) Application number: 01961505.3
(22) Date of filing: 20.08.2001
(51) Int. Cl.: A61F 13/64

(54) **ABSORBENT ARTICLE PROVIDED WITH A BELT**
SAUGFÄHIGER ARTIKEL MIT GÜRTEL
ARTICLE ABSORBANT POURVU D'UNE CEINTURE

(43) Date of publication of application: 02.06.2004
(73) Proprietor: SCA Hygiene Products AB, 405 03 Göteborg (SE)
(72) Inventor: HERMANSSON, Kent, S-426 54 Västra Frölunda (SE); STRANNEMALM, Kenneth, S-448 31 Floda (SE); KARLSSON, Katharina, S-411 95 Alingsas (SE)
(74) Representative: Egeröd, Lisbeth
(86) International application number: PCT/SE2001/001771
(87) International publication number: WO 2003/015685

(56) References cited:
- EP-A1- 0 993 817
- EP-A1- 1 142 547
- EP-A2- 1 077 054
- WO-A1-00/27330
- WO-A1-95/19753
- GB-A- 2 277 867
- US-A- 5 624 428
- US-A- 5 971 970

## Description

### Technical field

The present invention relates to an absorbent article, such as a diaper and an incontinence guard, comprising a liquid-pervious topsheet, a liquid-impervious backsheet and an absorbent body enclosed therebetween, wherein the article exhibits a front portion, a rear portion, and a crotch portion located therebetween, and further exhibits a belt, attached to or intended to be attached to the rear portion and to the front portion of the article, in such a way that the article assumes a pant-like shape where the belt constitutes a part of the waist portion of the pant.

### Background of the invention

Diapers and incontinence guards for incontinent adults usually have a garment portion holding an absorbent body in place against the user's body, and attachment means which hold the garment portion in place also when the user is moving. A common type of attachment means is adhesive tape tabs or hook and loop fasteners, which directly attach the front and rear portions of the absorbent article to each other.

It is also previously known, e.g. through EP-A-0 287 388, EP-A-0 409 307, EP-A-0 528 2282, EP-A-0 605 012 and FR-A-2 586 558, to attach the front and rear portions of the article to each other by means of a belt, wherein the possibilities to adjust the fit are imroved. Depending on the type of attachment means, different types of receiving surfaces, which are to cooperate with the attachment means, are utilised. Another advantage with belt diapers is that a user which is standing up, when putting the diaper on, first can fix the belt around his/her waist and then raise the absorbent portion of the diaper which is hanging down between his/her legs and fix its attachments means to the belt. In many cases, users having a reduced coordination or ability to move perceive such a dressing as being easier than it would be to put on a conventional "all-in-one" diaper or pant diaper.

Furthermore, WO 95/19753 discloses an absorbent article, such as a disposable diaper, an incontinence guard, a pant diaper or the like. The absorbent article comprises a containment arrangement comprising a liquid-pervious frontsheet, a liquid-impervious backsheet, an absorbent core arranged between the front- and backsheet, a first attachment arrangement and a second attachment arrangement. The attachment system disclosed in WO 95/19753 comprises that both the belt and the opposite waist portion of the diaper have attachment means on both the inside and the outside, and is in this way reported to enable the carrier to choose between putting on the article as a conventional diaper, as a pant diaper, or as a belt diaper. Furthermore, the attachment system is claimed to be designed for ensuring an easy removal and change of the diaper, and an easy inspection of whether the diaper has been soiled.

However, the previously known belt diapers can be perceived as being associated with certain problems.

Unlike infants, incontinent adults may have body sizes and shapes within a very wide range. The previously known diapers often have first attachment means of a limited area on one of the belt portions and second attachment means of a limited area on the other belt portion. Consequently, in order to obtain an attachment of the first attachment means to the second attachment means, it is required that the relatively small attachment areas of the first and second attachment means come into contact with each other. Besides resulting in demands on precision when fixating the belt, difficulties in adapting the belt length to different body sizes will arise. The previously known absorbent belt products often exhibit similar problems when locally delimited attachment means on the front portion of the diaper are to be attached to corresponding locally delimited attachment means on the belt in order to complete the dressing.

When nursing homes and the like are concerned, it sometimes occurs that incontinent adult users of belt diapers participate in toilet training or in the daytime more or less independently take care of their toilet visits. With the previously known belt diapers, there is a large risk that the portion of the diaper hanging down after having been released from the belt accidentally is dipped down into the toilet bowl when the user sits down or gets up. This is of course not desirable.

### Object and most important features of the invention

Accordingly, the object of the present invention is to achieve an absorbent article which eliminates the problem with the article accidentally being dipped down into a toilet bowl in connection with toilet training or a user's independent toilet visits, which article furthermore can be adapted to most occurring body sizes and shapes, and which article in addition optionally can be put on and taken off as a belt product, an "all-in-one" product, on in a similar fashion as a pant diaper.

In accordance with claim 1, this first object is achieved by means of an absorbent article, such as a diaper or an incontinence guard, which comprises a liquid-pervious topsheet, a liquid-impervious backsheet, and an absorbent body enclosed therebetween. The article exhibits a front portion, a rear portion, and a crotch portion located therebetween, and further a belt, attached to or intended to be attached to the rear portion and to the front portion of the article in such a way that the article assumes a pant-like shape where the belt constitutes a part of the waist portion of the pants. Thereby, at least a first belt portion carries a receiving material both on the face which is to constitute the outside and on the face which is to constitute the inside of the belt, which receiving material can serve as a receiving area for an attachment material arranged on a second belt portion and as a receiving area for an attachment material arranged on one or several attachment means attached to the front portion of the article which comprise a first main surface and an opposite, second main surface, and wherein the receiving surface also enables reattachment of the attachment material. According to the invention, the attachment material on the attachment means is arranged on both the first and the second main surface of at least one of the attachment means when it is in an unfolded position, wherein the belt portion or belt portions carry/carries the receiving material across substantially its/their entire outside and inside.

### Brief description of the drawings

In the following, the invention will be described in greater detail with reference to the attached drawings, in which
- Fig. 1: schematically shows a perspective view of an absorbent article according to a preferred embodiment of the invention, with a cut-out portion and seen from the face of the article which is intended to stand in contact with the skin of a user,
- Fig. 2: schematically, in a cross-sectional view through the line II-II in Fig. 1, shows the attachment means of the absorbent article, wherein one of the attachment means is in an unfolded position, whereas the other attachment means is in a folded-in position, and the thicknesses of the different material layers for reasons of clarity have been exaggerated,
- Fig. 3: schematically shows the absorbent article in Fig. 1 with its belt applied around the belt of a user,
- Fig. 4: schematically shows the absorbent article in Fig. 3 in a suspended position, which eliminates the problem of the article being accidentally dipped into a toilet bowl in connection with toilet training or a user's independent toilet visits,
- Fig. 5: schematically shows the absorbent article in Fig. 3 after having been completely dressed on the user as a belt product, and
- Fig. 6: schematically shows the absorbent article in Fig. 1 after having been completely dressed on the user as an "all-in-one" product, alternatively in a similar fashion as a pant diaper.

### Description of embodiments

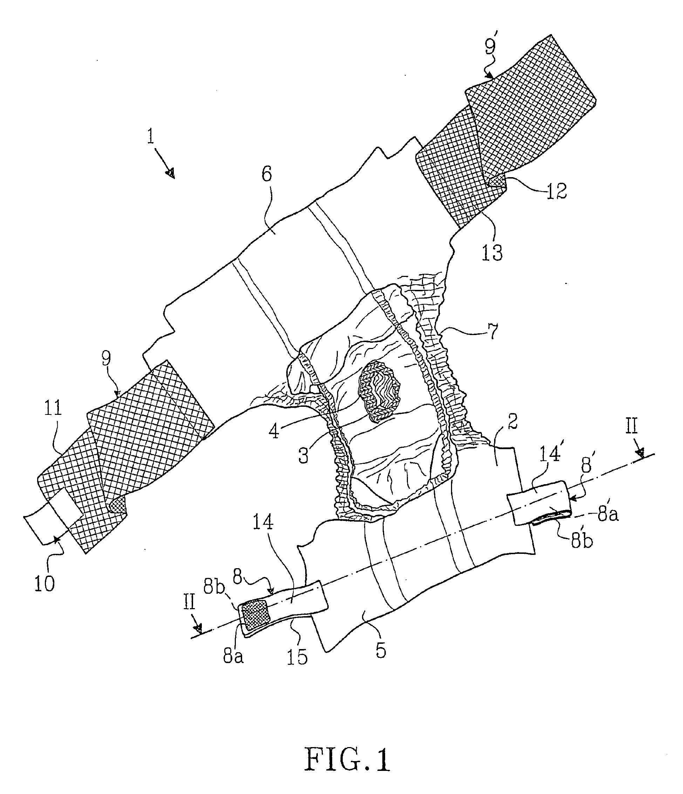

Fig. 1 shows a preferred embodiment of an absorbent article according to the invention in form of an incontinence diaper 1 comprising a liquid pervious topsheet 2, a liquid-impervious backsheet 3, and an absorbent body 4 enclosed therebetween. The liquid-pervious topsheet 2 can consist of a nonwoven material, e.g. a spunbond material of continuous filaments, a meltblown material, or a bonded, carded fibre material. The liquid-impervious backsheet 3 can consist of a plastic film, a nonwoven material coated with a liquid-arresting material, or a hydrophobic nonwoven material which resists liquid penetration.

The topsheet 2 and the backsheet 3 have a slightly larger extension in the plane than the absorbent body 4 and extend outside its edges. The layers 2 and 3 are mutually interconnected within the extending portions, for example by means of glueing or welding with heat or ultrasonic.

The absorbent body 4 can be of any conventional type. Examples of commonly occurring absorption materials are cellulose fluff pulp, tissue layers, highly absorptive polymers (socalled superabsorbents), absorbent foam materials, absorbent nonwoven materials, and the like. It is common to combine cellulose fluff pulp with superabsorbents in an absorbent body. It is also common with absorbent bodies constituted of layers of different materials having different properties when liquid acquisition ability, distribution ability and storage ability are concerned. This is well known to the skilled person and does not have to be described in detail. The thin absorbent cores which are common in for example baby diapers and incontinence guards often consist of a compressed mixed or layered structure of cellulose fluff pulp and superabsorbent.

The diaper is intended to enclose the lower portion of the torso of the user as a pair of absorbent pants. It exhibits a front portion 5 intended to be facing forwards on the user during use, a rear portion 6 intended to be facing backwards on the user during use, and a narrower crotch portion 7 located between the front and rear portion, which is intended to be applied in the crotch of the user between his/her legs.
A pair of belt portions 9, 9' are with one of their ends attached to, e.g. glued or ultrasonically welded to, the rear portion 6 of the diaper. The width of the belt portions 9, 9 is advantageously between 5-20 cm, preferably between 7-15 cm. On the face which is to constitute the outside and on the face which is to constitute inside of the belt, at least one of the belt portions 9' carries a receiving material 12, 13 which can serve as a receiving area for an attachment material 10 arranged on the other belt portion 9. Accordingly, the belt portions 9, 9' are intended to be attached to each other by means of the attachment material 10, for example a hook material, which is received on the opposite belt portion. Instead of hook material, optionally another attachment material can be used, such as adhesive tape.

The receiving material 12, 13 of the belt should also serve as a receiving area for an attachment material arranged on one or several attachment means 8,8' attached to the front portion 5 of the article. These attachment means 8, 8' comprise a first main surface, and an opposite, second main surface and are intended to be attached to the belt portions 9, 9' in order to hold together the diaper in the desired pant-like shape. The belt portions 9, 9' can be constituted for example of a laminate bonded in a suitable way of a carrier material 11 which on both sides is enclosed by nonwoven materials 12, 13. These nonwoven materials can be identical or different. However, it is also conceivable with embodiments where the belt portions are constituted of a single material layer which both on the outside and the inside of the belt is able to serve as a receiving material for attachment means, for example in the form of hook material and/or adhesive tape. Thus, the attachment means are partly the attachment material 10 on one of the belt portions 9 which is intended to be attached to the outside of the opposite belt portion 9', and partly the attachment means 8, 8' on the front portion of the diaper, which are intended to be attached to the belt 9, 9' for holding together the diaper in the desired pant-shape. Thereby, the receiving area has to enable both release and reattachment of the attachment material both on the other belt portion 9 and on the attachment means 8, 8'.

According to the invention, the attachment material 8a, 8b, 8'a, 8' is arranged on the attachment means 8, 8' on both the first and the second main surface of at least one of the attachment means when this is in an unfolded position, wherein the belt portion or the belt portions 9, 9' carries the receiving material 12, 13 across substantially the entire outside and inside. This provides an excellent adaptability to users with different body sizes and shapes, since preferably the entire outside and inside of the belt 9, 9' is able to serve as a receiving area for the attachment means 8, 8' and 10 enabling release and reattachment of the attachment means.

The attachment material 8a, 8b, 8'a, S'b of the attachment means 8, 8' is particularly advantageously constituted by a "hook material", but can also be constituted of adhesive tape or the like. As an attachment area for adhesive tape, usually a smooth or embossed plastic film is utilised, while nonwoven materials normally function well as ("loop material" and) and attachment area for the hook material. As has become evident in the foregoing, it also should be possible to release and reattach the attachment means. A nonwoven material as an attachment area for hook fasteners can advantageously consist of continuous filaments, such as a spunbond material or a meltblown material of, for example, polypropylene, polyethylene or bicomponent fibres.

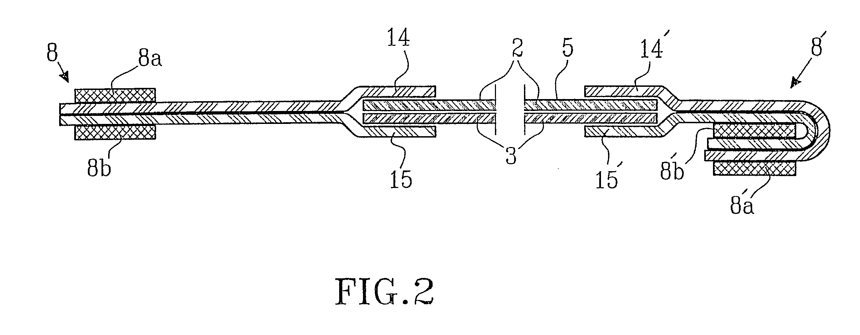

In a preferred embodiment of the absorbent article according to the invention, particularly illustrated in Fig. 2, one or several of the attachment means 8, 8' comprise(s) additional receiving material 15, 15' which can serve as a receiving area for the attachment material 8b, 8'b on either the first or second main surface of the attachment means 8, 8' when it is desired to bring the attachment means into a folded-in position. In the embodiment shown in Fig. 2, the material layers 14, 14' are a polyethylene film which, accordingly, advantageously can be replaced with an additional receiving material. Amongst other things, this embodiment provides the advantage that the attachment means 8, 8' can be brought into a folded-in position during transport and storage in order to minimise the surfaces of attachment material which are exposed and accidentally may attach to the receiving material of the absorbent article. In Fig. 2, the risk of the attachment material 8'b accidentally being attached to a receiving material of the absorbent article has been eliminated by means of folding in the attachment means 8' against the additional receiving material 15', while the attachment material 8'a still is exposed. In order to eliminate exposed attachment material completely during transport and storage of the absorbent article, a removable, protecting tape or the like (not shown in Fig. 2) can be applied on those attachment surfaces which still are exposed when the attachment means 8, 8' have been folded in.

It is also conceivable with embodiments (Fig. 1) where the outside and/or the inside of the belt 9, 9' serve(s) as a receiving area for different types of attachment means 8, 8' and 10, for example in such a way that the attachment means 8, 8' comprise hook material whereas the attachment material 10 is constituted of adhesive tape. At least the nonwoven material 13 which is to constitute the inside of the belt, i.e. be in direct contact with the skin of the user, should be soft an skin-friendly, for example a spun-bond material or a meltblown material of polypropylene or polyethylene, or a carded, thermobonded material. It is also conceivable with embodiments in which an elastic carrier material is included in the belt.

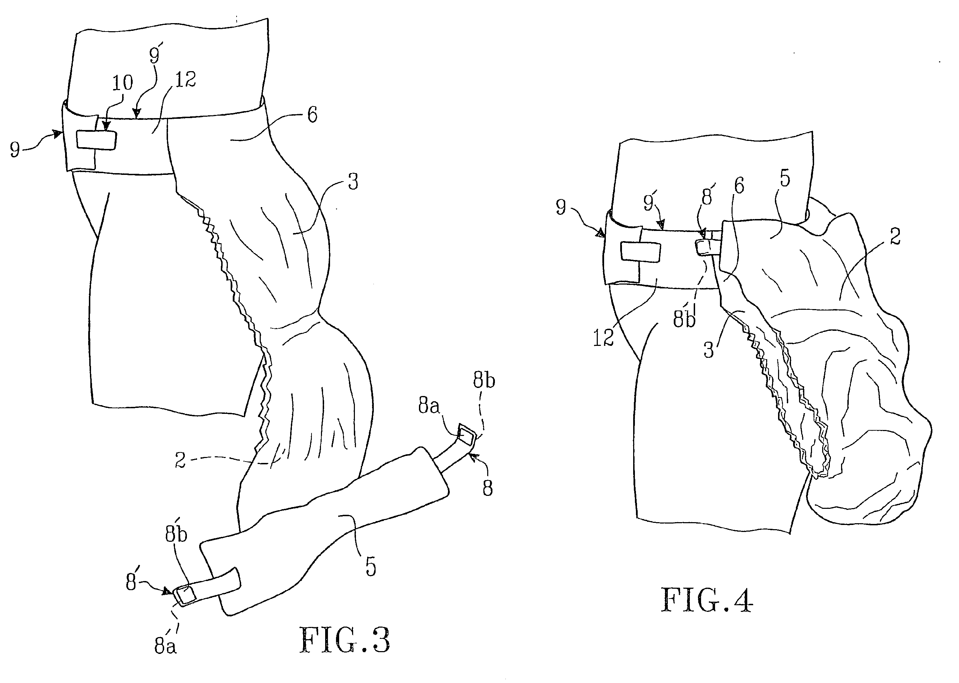

In a particularly preferred embodimnet, illustrated in Figs. 3 and 4, the attachment material on the second main surface 8'b is arranged so that, after the application of the belt 9, 9' on a user, the attachment material 8'b can be received by the receiving material 12 on the side which constitutes the outside of the belt 9' with the second main surface substantially in parallel to the plane of the receiving material 12 within the receiving area in order to suspend the front portion 5 of the article in the belt 9' with the front portion 5 on top of the rear portion 6 of the article. This embodiment provides the advantage that the attachment means 8' does not have to be turned around its axis or folded in order to be attached to the receiving material 12 of the belt in order to eliminate the problem with the article accidentally being dipped into a toilet bowl in connection with toilet training or a user's independent toilet visits.

In addition to making it easier for the user, in this embodiment a safer attachment of the attachment means 8, 8' to the receiving material 12 is obtained than what has been possible previously.

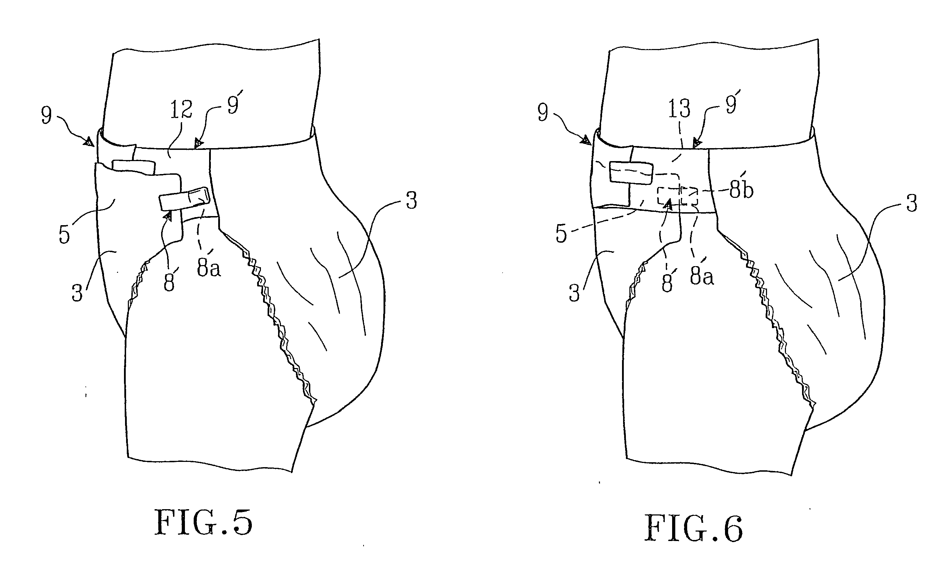

In one advantageous embodiment, particularly illustrated in Fig. 5, the attachment material on the first main surface 8'a is arranged so that, when putting the article on a user as a belt product, the attachment material 8'a can be received by the receiving material 12 on the face which is to constitute the outside of the belt 9' with the first main surface substantially parallel to the plane of the receiving material 12 within the receiving area.

In another advantageous embodiment, particularly illustrated in Fig. 6, the attachment material on the second main surface 8'b is arranged so that, when putting the article on a user as an all-in-one product, the attachment material 8'b can be received by the receiving material 13 on the side which is to constitute the inside of the belt 9' with the second main surface substantially parallel to the plane of the receiving material 13 within the receiving area. This embodiment advantageously can be utilised when a user or nursing staff for some reason prefer application as an all-in-one product to application as a belt product.

In still another advatnageous embodiment, not explicitly shown but still described with reference to Fig. 6, at least one of the attachment means 8' is in the folded-in position with the attachment material on the second main surface 8'b received by the additional receiving material 15', wherein the attachment material on the first main surface 8'a is arranged so that, when putting the article on a user in a similar fashion as a pant diaper, the attachment material on the first main surface 8'a can be received by the receiving material 13 on the face which is to constitute the inside of the belt 9'. Since the attachment means 8, 8', due to their being folded-in, gets a reduced length and flexibility, this embodiment enables the front portion 5 of the absorbent article to be retained in a particularly stable way beneath the belt 9, 9' in those cases when the absorbent article already before being put on is to be attached in a pant diaper-like fashion.

The invention is of course not limited to the above-described embodiments, but can be modified within the scope of the claims.

## Claims

1. An absorbent article, such as a diaper and an incontinence guard, comprising a liquid-pervious topsheet (2), a liquid-impervious backsheet (3), and an absorbent body (4) enclosed therebetween, wherein the article exhibits a front portion (5), a rear portion (6) and a crotch portion (7) located therebetween, and further exhibits a belt (9,9') attached to or intended to be attached to the rear portion (6) and to the front portion (5) of the article in such a way that the article assumes a pant-like shape where the belt (9,9') constitutes a part of the waist portion of the pant, wherein at least a first belt portion (9') both on the face which is to constitute the outside and on the face which is to constitute the inside of the belt carries a receiving material (12, 13), which can serve as a receiving area for an attachment material (10) arranged on a second belt portion (9) and as a receiving area for an attachment material arranged on one or several attachment means (8, 8'), attached to the front portion (5) of the article, comprising a first main surface and an opposite, second main surface, and wherein said receiving material (12, 13) also enables reattachment of said attachment material, **characterised in that** the attachment material (8a, 8b, 8'a, 8'b) on said attachment means (8, 8') is arranged on both the first and the second main surface of at least one of said attachment means when it is in an unfolded position, and that said belt portion or belt portions (9,9') carry/carries said receiving material (12,13) across substantially its/their entire outside and inside.

2. An absorbent article according to claim 1,
**characterised in that** one or several of said attachment means (8, 8') comprise(s) additional receiving material (15, 15') which can serve as a receiving area for said attachment material (8b, 8'b) either on the first or second main surface of the attachment means (8, 8') when it is desired to bring said attachment means into a folded-in position.

3. An absorbent article according to claim 1 or 2,
**characterised in that** the attachment material on the second main surface (8'b) is arranged so that, after applying the belt (9, 9') onto a user, said attachment material (8'b) can be received by the receiving material (12) on the face constituting the outside of the belt (9') with said second main surface substantially iparallel to the plane of said receiving material (12) within said receiving area in order to suspend the front portion (5) of the article in said belt (9') with said front portion (5) on top of the rear portion (6) of said article.

4. An absorbent article according to any one of claims 1, 2 or 3,
**characterised in that** the attachment material on the first main surface (8'a) is arranged so that, when putting the article on a user as a belt product, said attachment material (8'a) can be received by the receiving material (12) on the face which is to constitute the outside of the belt (9') with said first main surface substantially parallel to the plane of said receiving material (12) within said receiving area.

5. An absorbent article according to any one of claims 1-4,
**characterised in that** the attachment material on the second main surface (8'b) is arranged so that, when putting the article on a user as an all-in-one product, said attachment material (8'b) can be received by the receiving material (13) on the face which is to constitute the inside of the belt (9') with said second main surface substantially parallel to the plane of said receiving material (13) within said receiving area.

6. An absorbent article according to any one of claims 2 - 4,
**characterised in that** at least one of the attachment means (8') is in the folded-in position with the attachment material on the second main surface (8'b) received by the additional receiving material (15'), wherein the attachment material on the first main surface (8'a) is arranged so that, when putting the article on a user in a similar fashion as a pant diaper, said attachment material on the first mains surface (8'a) can be received by the receiving material (13) on the face which is to constitute the inside of the belt (9').

7. An absorbent article according to any one of the preceding claims,
**characterised in that** the attachment material (8a, 8'a, 8b, 8'b, 10) of said belt (9, 9') and/or of said attachment means (8,8') is constituted of hook material or adhesive tape.

8. An absorbent article according to any one of the preceding claims,
**characterised in that** the receiving material (12, 13) of said belt (9, 9'), and/or that the additional receiving material (15, 15') of said attachment means (8, 8'), primarily is made of continuous filaments, such as a spunbond or meltblown material.

## Patentansprüche

1. Absorbensartikel, wie zum Beispiel eine Windel und ein Inkontinenzschutz, umfassend eine flüssigkeitsdurchlässige Oberlage (2), eine flüssigkeitsundurchlässige Rücklage (3) und einen Absorbenskörper (4), der dazwischen eingeschlossen ist, wobei der Artikel einen Frontabschnitt (5), einen Rückabschnitt (6) und einen Schrittabschnitt (7), der dazwischen angeordnet ist, aufweist, und ferner einen Gürtel (9, 9') aufweist, der derart an dem Rückabschnitt (6) und an dem Frontabschnitt (5) des Artikels angebracht ist oder dazu gedacht ist, dort angebracht zu sein, dass der Artikel eine hosenähnliche Form annimmt, bei welcher der Gürtel (9, 9') einen Teil des Taillenabschnitts der Hose bildet, wobei zumindest ein erster Gürtelabschnitt (9') sowohl auf der Fläche, welche die Außenseite des Gürtels bildet, als auch auf der Fläche, welche die Innenseite bildet, ein Aufnahmematerial (12, 13) trägt, das als Aufnahmefläche für ein Befestigungsmaterial (10), das an einem zweiten Gürtelabschnitt (9) angeordnet ist, und als Aufnahmefläche für ein Befestigungsmaterial, das an einem oder mehreren Befestigungsmitteln (8, 8') angeordnet ist, die an dem Frontabschnitt (5) des Artikels angebracht sind, dienen kann, umfassend eine erste Hautoberfläche und eine gegenüberliegende zweite Hauptoberfläche, und wobei das Aufnahmematerial (12, 13) auch ein Niederbefestigen des Befestigungsmaterials ermöglicht,
**dadurch gekennzeichnet, dass**
das Befestigungsmaterial (8a, 8b, 8'a, 8'b) an den Befestigungsmitteln (8, 8') sowohl auf der ersten als auch auf der zweiten Hauptoberfläche von zumindest einem der Befestigungsmittel angeordnet ist, wenn es in einer entfalteten Position ist, und dass der Gürtelabschnitt oder die Gürtelabschnitte (9, 9') das Aufnahmematerial (12, 13) im Wesentlichen über seine/ihre gesamte/gesamten Außenseite/Außenseiten und Innenseite/Innenseiten trägt/tragen.

2. Absorbensartikel nach Anspruch 1, **dadurch gekennzeichnet, dass** eins oder mehrere der Befestigungsmittel (8, 8') zusätzliches Aufnahmematerial (15, 15') aufweist/aufweisen, das als eine Aufnahmefläche für das Befestigungsmaterial (8b, 8'b) entweder an der ersten oder der zweiten hauptoberfläche der Gefestigungsmitte (8, 8') dienen kann, wenn es gewünscht ist, die Befestigungsmittel in eine eingefaltete Position zu bringen.

3. Absorbensartikel nach Anspruch 1 oder 2, **dadurch gekennzeichnet, dass** das Befestigungsmaterial an der zweiten Hauptoberfläche (8'b) so angeordnet ist, dass das Befestigungsmaterial (8'b) nach dem Anbringen des Gürtels (9, 9') an einem Benutzer durch das Aufnahmematerial (12) an der fläche, welche die Außenseite des Gürtels (9') bildet, aufgenommen werden kann, wobei die zweite Hauptoberfläche im Wesentlichen parallel zu der Fläche des Aufnahmematerials (12) innerhalb der Aufnahmefläche ist, um den Frontabschnitt (5) des Artikels in dem Gürtel (9') mit dem Frontabschnitt (5) auf dem Rückabschnitts (6) des Artikels aufzuhängen.

4. Absorbensartikel nach einem der Ansprüche 1, 2 oder 3, **dadurch gekennzeichnet, dass** das Befestigungsmaterial an der ersten Hauptoberfläche (8'a) derart angeordnet ist, dass, wenn der Artikel einem Benutzer als ein Gürtelprodukt angezogen wird, das Befestigungsmaterial (8'a) durch das Aufnahmematerial (12) an der Fläche, welche die Außenseite des Gürtels (9') bildet, aufgenommen werden kann, wobei die erste Hauptoberfläche im Wesentlichen parallel zu der Ebene des Aufnahmematerials (12) innerhalb der Aufnahmefläche ist.

5. Absorbensartikel nach einem der Ansprüche 1 bis 4, **dadurch gekennzeichnet, dass** das Befestigungsmaterial an der zweiten Hauptoberfläche (8'b) derart angeordnet ist, dass das Befestigungsmaterial (8'b) von dem Aufnahmematerial (13) an der Fläche aufgenommen werden kann, welche die Innenseite des Gürtels (9') bildet, wenn der Artikel einem Benutzer als ein einteiliges Produkt angezogen wird, wobei die zweite Hauptoberfläche im Wesentlichen parallel zu der Ebene des Aufnahmematerials (13) innerhalb der Aufnahmefläche ist.

6. Absorbensartikel nach einem der Ansprüche 2 bis 4, **dadurch gekennzeichnet, dass** zumindest eins der Befestigungsmittel (8') in der eingefalteten Position ist, wobei das Befestigungsmaterial an der zweiten Hauptoberfläche (8'b) durch das zusätzliche Aufnahmematerial (15') aufgenommen ist, wobei das Befestigungsmaterial an der ersten Hauptoberfläche (8'a) so angeordnet ist, dass das Befestigungsmaterial an der ersten Hauptoberfläche (8'a) von dem Aufnahmematerial (13) an der Fläche aufgenommen werden kann, welche die Innenseite des Gürtels (9') bildet, wenn der Artikel einem Benutzer auf eine ähnliche Weise wie eine Höschenwindel angezogen wird.

7. Absorbensartikel nach einem der vorstehenden Ansprüche, **dadurch gekennzeichnet, dass** das Befestigungsmaterial (8a, 8'a, 8b, 8'b, 10) des Gürtels (9, 9') und/oder der Befestigungsmittel (8, 8') aus Hakenmaterial oder Klebeband gebildet ist.

8. Absorbensartikel nach einem der vorstehenden Ansprüche, **dadurch gekennzeichnet, dass** das Aufnahmematerial (12, 13) des Gürtels (9, 9') und/oder dass das zusätzliche Aufnahmematerial (15, 15') der Befestigungsmittel (8, 8') hauptsächlich aus durchgehenden Filamenten, wie zum Beispiel einem Spunbond oder einem Meltblown Material gemacht ist.

## Revendications

1. Article absorbant, tel qu'une couche-culotte ou une protection contre l'incontinence, comprenant une feuille supérieure (2) perméable aux liquides, une feuille arrière (3) imperméable aux liquides et un corps absorbant (4) enfermé entre celles-ci, cet article présentant une partie avant (5), une partie arrière (6) et une partie entrejambe (7) située entre ces dernières et présentant en outre une ceinture (9, 9') fixée ou destinée à être fixée à la partie arrière (6) de l'article et à la partie avant (5) de l'article, et à la partie avant (5) de l'article, de telle manière que l'article prend une forme analogue à celle d'une culotte dans laquelle la ceinture (9, 9') constitue une portion de la partie taille de la culotte, au moins une première partie ceinture (9') aussi bien sur la face qui est destinée à constituer l'extérieur que sur la face qui est destinée à constituer l'intérieur de la ceinture, porte un matériau récepteur (12, 13) qui peut servir de surface de réception pour un matériau de fixation (10) agencé sur une deuxième partie ceinture (9) et de surface de réception pour un matériau de fixation (10) agencé sur un ou plusieurs moyens de fixation (8, 8') fixé(s) sur la partie avant (5) de l'article, comprenant une première surface principale et une deuxième surface principale, opposée et ledit matériau récepteur (12, 13) permettant également de refixer ledit matériau de fixation, **caractérisé en ce que** le matériau de fixation (8a, 8b, 8'a, 8'b) présent sur ledit moyen de fixation (8, 8') est agencé sur les deux surfaces principales, la première et la deuxième, d'au moins un desdits moyens de fixation lorsqu'il est en position non repliée et **en ce que** ladite partie ceinture ou lesdites parties ceinture (9, 9') porte/portent ledit matériau de réception (12, 13) sur substantiellement l'ensemble de sa/leur face extérieure et de sa/leur face intérieure.

2. Article absorbant selon la revendication 1,
**caractérisé en ce que** un ou plusieurs desdits moyens de fixation (8, 8') comprend/comprennent un matériau récepteur additionnel (15, 15') qui peut servir de surface de réception pour ledit matériau de fixation (8b, 8'b) sur l'une ou l'autre des première et deuxième surfaces principales du moyen de fixation (8, 8') lorsque l'on désire mettre ledit moyen de fixation en position repliée vers l'intérieur.

3. Article absorbant selon la revendication 1 ou 2,
**caractérisé en ce que** le moyen de fixation présent sur la deuxième surface principale (8'b) est agencé de telle manière qu'après la mise en place de la ceinture (9, 9') sur un utilisateur, ledit moyen de fixation (8'b) peut être reçu par le moyen de réception (12) présent sur la face constituant l'extérieur de la ceinture (9'), ladite deuxième surface principale étant substantiellement parallèle au plan dudit matériau récepteur (12) à l'intérieur de ladite surface de réception afin de suspendre la partie avant (5) de l'article dans ladite ceinture (9'), ladite partie avant (5) étant sur le haut de la partie arrière (6) dudit article.

4. Article absorbant selon l'une quelconque des revendications 1, 2 ou 3,
**caractérisé en ce que** le matériau de fixation présent sur la première surface principale (8'a) est agencé de telle manière que lorsque l'on met l'article en place sur un utilisateur en tant qu'article de ceinture, ledit matériau de fixation (8'a) peut être reçu par le matériau récepteur (12) présent sur la face qui est destinée à constituer l'extérieur de la ceinture (9'), ladite première surface principale étant substantiellement parallèle au plan dudit matériau récepteur (12) présent dans ladite surface de réception.

5. Article absorbant selon l'une quelconque des revendications 1 à 4,
**caractérisé en ce que** le matériau de fixation présent sur la deuxième surface principale (8'b) est agencé de telle manière que lorsque l'on met en place l'article sur un utilisateur en tant que produit réalisé en une seule pièce, ledit matériau de fixation (8'b) peut être reçu par le matériau récepteur (13) présent sur la face qui est destinée à constituer l'intérieur de la ceinture (9'), ladite deuxième surface principale étant substantiellement parallèle au plan dudit matériau récepteur (13) à l'intérieur de ladite surface de réception.

6. Article absorbant selon l'une quelconque des revendications 2 à 4,
**caractérisé en ce qu'**au moins un des moyens de fixation (8') est en position repliée, le matériau de fixation présent sur la deuxième surface principale (8'b) étant reçu par le matériau récepteur additionnel (15'), le matériau de fixation présent sur la première surface principale (8'a) étant agencé de telle manière que lorsque l'on met en place l'article sur un utilisateur d'une manière similaire à une couche-culotte, ledit matériau de fixation présent sur la première surface principale (8'a) peut être reçu par le matériau récepteur (13) présent sur la face qui est destinée à constituer l'intérieur de la ceinture (9').

7. Article absorbant selon l'une quelconque des revendications qui précèdent,
**caractérisé en ce que** le matériau de fixation (8a, 8'a, 8b, 8'b, 10) de ladite ceinture (9, 9') et/ou dudit moyen de fixation (8, 8') est constitué par un matériau du type à crochets ou par un ruban adhésif.

8. Article absorbant selon l'une quelconque des revendications qui précèdent,
**caractérisé en ce que** le matériau récepteur (12, 13) de ladite ceinture (9, 9') et/ou le matériau récepteur additionnel (15, 15') dudit moyen de fixation (8, 8') est/sont principalement constitué(s) de filaments continus, tel qu'un matériau lié au filage ou un matériau soufflé fondu.
